# EUROPEAN PATENT SPECIFICATION

(11) **EP 4 393 373 B1**
(45) Date of publication and mention of the grant of the patent: **30.07.2025**
(21) Application number: 22938233.8
(22) Date of filing: 17.10.2022
(51) Int. Cl.: A61B 1/00, A61B 1/005

(54) **ENDOSCOPE CONNECTING STRUCTURE, ENDOSCOPE HANDLE, AND ENDOSCOPE**
ENDOSKOPVERBINDUNGSSTRUKTUR, ENDOSKOPGRIFF UND ENDOSKOP
STRUCTURE DE RACCORDEMENT D'ENDOSCOPE, POIGNÉE D'ENDOSCOPE ET ENDOSCOPE

(30) Priority: 22.04.2022 CN 202210425083
(43) Date of publication of application: 03.07.2024
(73) Proprietor: Hunan Vathin Medical Instrument Co. Ltd, Xiangtan, Hunan 411201 (CN)
(72) Inventor: ZHOU, Zhenhua, Xiamen, Fujian 361000 (CN)
(74) Representative: Bayramoglu et al.
(86) International application number: PCT/CN2022/125754
(87) International publication number: WO 2023/202010

(56) References cited:
- WO-A1-2021/145051
- WO-A2-2014/183853
- CN-A- 107 320 057
- CN-A- 114 052 624
- CN-A- 114 748 020
- CN-U- 207 912 660
- CN-U- 217 138 010
- US-A- 4 294 233
- US-A- 4 941 454
- US-A1- 2022 330 790
- US-A9- 2020 121 169

## Description

### TECHNICAL FIELD

The present disclosure relates to the technical field of endoscopy, and in particular to an endoscope connecting structure, an endoscope handle, and an endoscope.

### BACKGROUND

As a commonly used medical examination instrument, an endoscope can directly enter the natural orifice of human body, providing the doctor with sufficient information for diagnosis and treatment. When in use, the inserting unit of the endoscope enters the human body through a natural orifice or a small surgical incision, such that the doctor can directly observe changes in relevant parts.

In the prior art, after the upper and lower handles of a detachable endoscope are connected together, a large amount of space is required to provide the range of movement for the traction wires when the traction wires are driven. The design reduces the space required for other components in the endoscope handle where the traction wires are located, thereby affecting the normal operation of other components. US4294233A discloses a slack absorbing device in the bending operation mechanism of an endoscope. Slack absorbing members are moved along the passage ways of connecting wires as the bending section is bent. Each slack absorbing member has a long groove extending along the direction of movement of said slack absorbing member and a threaded groove formed in a portion of the long groove. Threaded rods connect the ends of the wires extending to the bending section provided in the top end part of the endoscope in such a manner that the threaded rods are moved together with the wires at least when the wires are pulled towards the manual operating section. A part of each threaded groove is cut off so that the threaded rod can engage the threaded groove by aligning the threaded rod, in a direction corresponding to the direction of movement of the wire, and placing it into the groove.

### SUMMARY

An objective of the present disclosure is to provide an endoscope connecting structure, an endoscope handle, and an endoscope. The present disclosure aims to solve the above-mentioned technical problems existing in the prior art, and includes the following three aspects.

A first aspect of the present disclosure provides an endoscope connecting structure, including:
an accommodating housing, provided therein with an accommodating space;
a transmission element, slidably provided in the accommodating space;
traction wire mounting elements, respectively provided at two ends of the transmission element and located in the accommodating space; and
traction wires, where one end of each of the traction wires is fixedly connected to a corresponding one of the traction wire mounting elements.

The accommodating housing is U-shaped, V-shaped or W-shaped and provided with a hollow structure; the hollow structure of the accommodating housing forms the accommodating space; the transmission element is slidable and rollable along an inner wall of the accommodating space

Each of the two arms of the accommodating housing is provided at its external side with a wire passage; and the traction wires are respectively connected to the traction wire mounting elements through the wire passages.

Further, preferably, the transmission element is either a solid or a viscous liquid; and a viscosity of the viscous liquid is greater than a preset viscosity.

Further, preferably, the two ends of the transmission element are not higher than the two ends of the accommodating housing.

Further, preferably, an inner wall of the accommodating housing is provided with a locking groove.

Further, preferably, limiting portions are respectively provided at the two ends of the accommodating housing.

A second aspect of the present disclosure provides an endoscope handle, including:
an upper handle, including a pushing assembly; and
a lower handle, including the above-mentioned endoscope connecting structure, where
one end of the pushing assembly is located in the accommodating space and abutted against the traction wire mounting elements.

Further, preferably, the pushing assembly includes at least a first pushing element, a second pushing element, and a driving element;
one end of the first pushing element is abutted against the traction wire mounting elements, and one end of the second pushing element is abutted against the traction wire mounting elements; and
the driving element is configured to drive the first pushing element and the second pushing element to move in opposite directions synchronously.

Further, preferably, a locking element matched with the locking groove is provided on an outer wall of the first pushing element and/or an outer wall of the second pushing element.

A third aspect of the present disclosure provides an endoscope, including the above-mentioned endoscope handle.

Compared with the prior art, the present disclosure has at least the following technical effects.
(1) In the endoscope connecting structure provided by the present disclosure, the first traction wire mounting element or the second traction wire mounting element is pushed by an external force to cause the transmission element to move in one direction along an inner wall of the accommodating housing, thereby driving the corresponding traction wire to move. In the present application, the movable range of the transmission element is determined by the shape and size of the accommodating space. That is, the transmission element is slidable along a preset path and space of the accommodating housing, without affecting the arrangement of other components during the sliding process. It can be understood that during the movement process, the movement of the transmission element will not affect the movement or arrangement of other components located on an outer wall of the accommodating housing. The present disclosure reduces the movement space of the transmission element and reduces the corresponding structure of an endoscope housing that is matched with the transmission element.
(2) In the present application, the accommodating housing is provided with the wire passages. When the transmission element moves, the traction wires are movable along the wire passages to ensure that the traction wires are always located outside the accommodating housing, avoiding the traction wires from entering the accommodating housing and affecting the movement of the transmission element.
(3) In the endoscope handle provided by the present application, the pushing assembly adopts a tooth engagement pushing method to achieve synchronous movement of the first pushing element and the second pushing element. When one pushing element moves down a preset distance, the other pushing element synchronously moves up an equal preset distance. In this way, the operator can control the movement distance of the traction wire by controlling the movement distance of the pushing element, in order to accurately control the traction wire.

### BRIEF DESCRIPTION OF THE DRAWINGS

To describe the technical solutions in the embodiments of the present disclosure more clearly, the following briefly introduces the drawings required for describing the embodiments or the prior art. Apparently, the drawings in the following description show merely some embodiments of the present disclosure, and persons of ordinary skill in the art may still derive other drawings from these drawings without creative efforts.
FIG. 1 is a schematic diagram of an endoscope connecting structure according to the present disclosure;
FIG. 2 is a sectional view of the endoscope connecting structure according to the present disclosure;
FIG. 3 is a structural diagram of an accommodating housing according to the present disclosure;
FIG. 4 is a structural diagram of a locking groove according to the present disclosure;
FIG. 5 is a structural diagram of a traction wire mounting element according to the present disclosure;
FIG. 6 is a first sectional view of the traction wire mounting element according to the present disclosure;
FIG. 7 is a second sectional view of the traction wire mounting element according to the present disclosure;
FIG. 8 is a structural diagram of an endoscope handle according to the present disclosure;
FIG. 9 is a structural diagram of a pushing assembly and a connecting structure that are connected according to the present disclosure; and
FIG. 10 is a sectional view of the pushing assembly and the connecting structure that are connected according to the present disclosure.

Reference Numerals: 2. lower handle; 21. housing; 211. first wire passage; 212. second wire passage; 22. transmission element; 23. first traction wire mounting element; 231. threading hole; 2311. first threading hole; 2312. second threading hole; 27. first expansion portion; 24. second traction wire mounting element; 241. threading hole; 2411. first threading hole; 2412. second threading hole; 28. second expansion portion; 26. locking groove; 31. first traction wire; 32. second traction wire; 10. pushing assembly; 11. first pushing element; 12. driving element; and 13. second pushing element.

### DETAILED DESCRIPTION OF THE EMBODIMENTS

The following description provides many different embodiments or examples for implementing different features of the present disclosure. The elements and arrangements described in the following specific examples are only intended to concisely express the present disclosure, and are only for illustration purposes, rather than to limit the present disclosure.

In order to make the objectives, technical solutions, and advantages of the embodiments of the present disclosure clearer, the technical solutions in the embodiments of the present disclosure will be clearly and completely described below in conjunction with the accompanying drawings in the embodiments of the present disclosure. Obviously, the described embodiments are some, rather than all of the embodiments of the present disclosure. On the basis of the embodiments of the present disclosure, all other embodiments obtained by a person of ordinary skill in the art without making creative efforts shall fall within the protection scope of the present disclosure. Therefore, the detailed description of the embodiments of the present disclosure in the accompanying drawings is not intended to limit the protection scope of the present disclosure, but merely represent the selected embodiments of the present disclosure. On the basis of the embodiments of the present disclosure, all other embodiments obtained by a person of ordinary skill in the art without making creative efforts shall fall within the protection scope of the present disclosure.

In the present disclosure, unless otherwise clearly specified, the terms "installation", "interconnection", "connection" and "fixation" etc. are intended to be understood in a broad sense. For example, the "connection" may be a fixed connection, removable connection or integral connection; may be a mechanical connection or electrical connection; may be a direct connection or indirect connection using a medium; and may be a communication or interaction between two elements. Those of ordinary skill in the art may understand specific meanings of the above terms in the present disclosure based on a specific situation. In addition, the terms such as "first", "second", and "third" are used only for the purpose of description and cannot be understood to indicate or imply relative importance.

### Embodiment 1

Embodiment 1 of the present disclosure provides an endoscope connecting structure, including: accommodating housing 21, transmission element 22, traction wire mounting elements 23 and 24, and traction wires 31 and 32.

The accommodating housing 21 is provided therein with an accommodating space.

The transmission element 22 is slidably provided in the accommodating space.

The traction wire mounting elements 23 and 24 are respectively provided at two ends of the transmission element 22, and are located in the accommodating space.

One end of each of the traction wires 31 and 32 is fixedly connected to a corresponding one of the traction wire mounting elements 23 and 24.

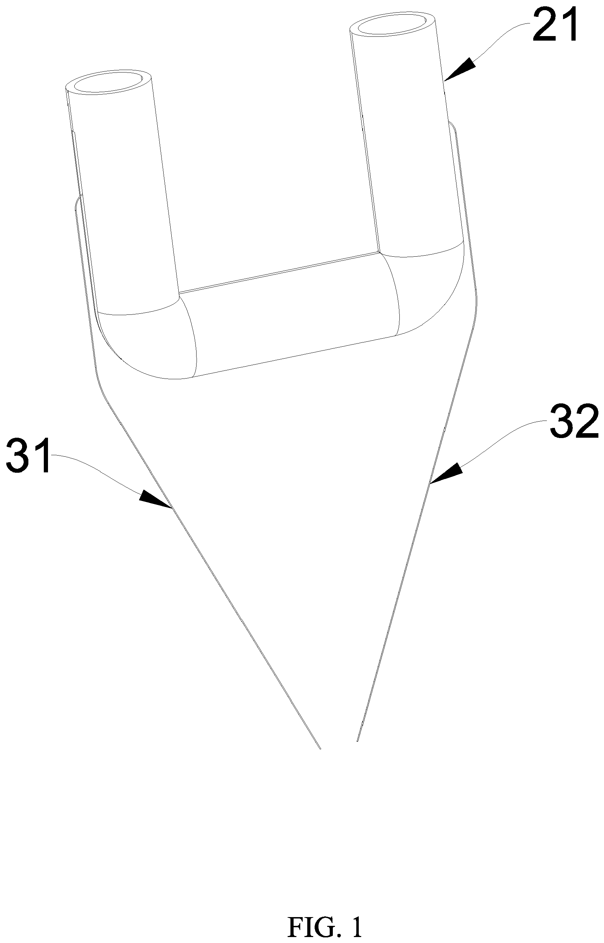

In the above solution, as shown in FIGS. 1 to 4, the accommodating housing 21 is U-shaped and provided with a hollow structure. The hollow structure of the accommodating housing 21 forms the accommodating space. The transmission element 22 is provided in the accommodating space. The transmission element 22 is slidable and rollable along an inner wall of the accommodating space. The two ends of the transmission element 22 are respectively provided with first traction wire mounting element 23 and second traction wire mounting element 24. Sides of the first traction wire mounting element 23 and the second traction wire mounting element 24 are respectively in contact with sides of corresponding ends of the transmission element 22 close to two ends of the accommodating housing 21. The contact is not in form of fixed or detachable connection. The contact can be point contact, line contact or surface contact, which is not limited herein. The first traction wire mounting element 23 is fixedly connected to first traction wire 31, and the second traction wire mounting element 24 is fixedly connected to second traction wire 32. The fixed connection can be achieved by means of adhesive bonding or integrated molding, which is not limited herein.

It should be noted that the accommodating housing 21 can also be V-shaped or W-shaped. The present application does not limit the shape of the accommodating housing 21, as long as the accommodating housing 21 can be provided in a matched component.

The traction wire mounting elements 23 and 24 include the first traction wire mounting element 23 and the second traction wire mounting element 24.

The traction wires 31 and 32 include the first traction wire 31 and the second traction wire 32.

It should be noted that in the present application, four traction wires can also be provided to achieve movement in other directions, that is, to provide two endoscope connecting structures. The two endoscope connecting structures can be the same or different and are at an angle. In the present application, the number, position, and connection relationship of the connecting structures are adjustable as needed to achieve the movement of multiple traction wires.

Therefore, in the endoscope connecting structure provided by the present disclosure, the first traction wire mounting element 23 or the second traction wire mounting element 24 is pushed by an external force to cause the transmission element 22 to move in one direction along an inner wall of the accommodating housing 21, thereby driving the corresponding traction wire to move. In the present application, the movable range of the transmission element 22 is determined by the shape and size of the accommodating space. That is, the transmission element 22 is slidable along a preset path and space of the accommodating housing 21, without affecting the arrangement of other components during the sliding process. It can be understood that during the movement process, the movement of the transmission element 22 will not affect the movement or arrangement of other components located on an outer wall of the accommodating housing 21. The present disclosure reduces the movement space of the transmission element 22 and reduces the corresponding structure of an endoscope housing that is matched with the transmission element 22.

Further, two sides of the accommodating housing 21 are respectively provided with wire passages 211 and 212. The traction wires 31 and 32 are respectively connected to the traction wire mounting elements 23 and 24 through the wire passages 211 and 212.

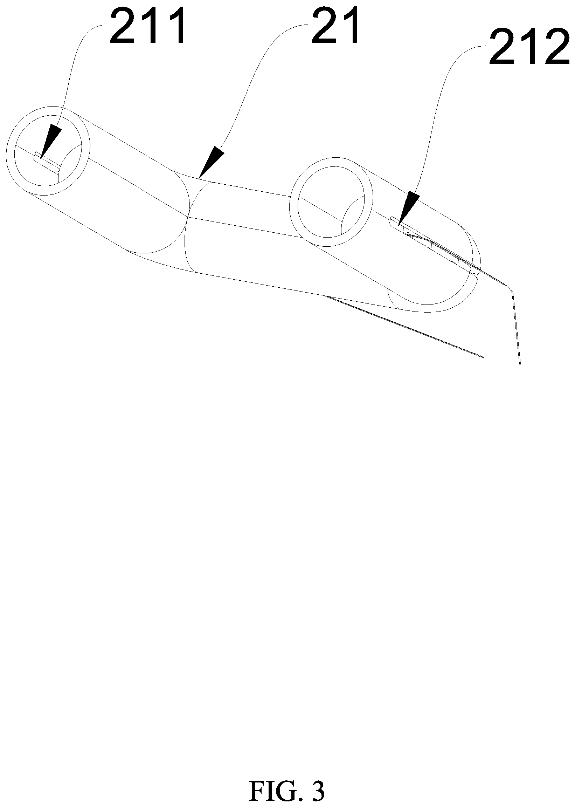

In the above solution, as shown in FIG. 3, the two sides of the accommodating housing 21 are respectively provided with the first wire passage 211 and the second wire passage 212. The first wire passage 211 and the second wire passage 212 are symmetrically arranged along an axis of symmetry of the accommodating housing 21, and can also be asymmetrically arranged. In the present application, preferably, the first wire passage 211 and the second wire passage 212 are symmetrically arranged. Sizes of the first wire passage 211 and the second wire passage 212 can be set according to thicknesses of the first traction wire 31 and the second traction wire, or according to a material of the transmission element 22 in the accommodating housing 21. When the transmission element 22 is solid, the first wire passage 211 and the second wire passage 212 can be wide to prevent the transmission element 22 from falling out of the accommodating housing 21 and reduce the weight of the accommodating housing 21. When the transmission element 22 is a viscous liquid, the first wire passage 211 and the second wire passage 212 can be narrow to allow only the corresponding traction wire to pass through. In the field of endoscopy, the diameter of the traction wire is very small. Therefore, the first wire passage 211 and the second wire passage 212 are very narrow. When the transmission element 22 is pushed by a pushing assembly, the transmission element 22 will not be squeezed out from the first wire passage 211 and the second wire passage 212.

It should be noted that in the present application, preferably, the wire passage is in the shape of a long strip. In addition, the wire passage can also be circular, square, triangular, etc., which is not limited herein.

In the present application, the accommodating housing 21 is provided with the wire passages. When the transmission element 22 moves, the traction wires are movable along the wire passages to ensure that the traction wires are always located outside the accommodating housing 21, avoiding the traction wires from entering the accommodating housing 21 and affecting the movement of the transmission element 22.

The wire passages 211 and 212 include the first wire passage 211 and the second wire passage 212.

Further, the transmission element 22 can be either a solid or a viscous liquid, and a viscosity of the viscous liquid is greater than a preset viscosity.

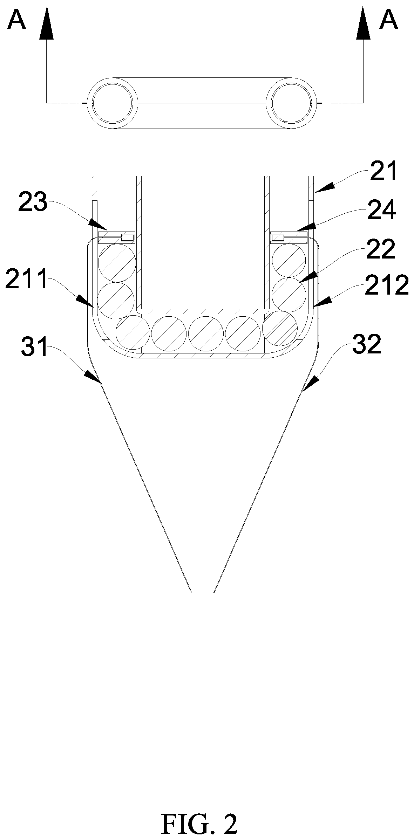

In the above solution, as shown in FIG. 2, the transmission element 22 can be a spherical solid, such as a solid made of iron and steel. The transmission element 22 can also be a viscous liquid, such as viscous butter. The viscosity of the viscous liquid is greater than the preset viscosity to ensure that the transmission element 22 slides normally in the accommodating housing 21. The transmission element 22 can also be made of silicone or other materials.

Further, the two ends of the transmission element 22 are not higher than the two ends of the accommodating housing 21.

In the above solution, the transmission element 22 can be pushed by the traction wire mounting elements 23 and 24 to slide inside the accommodating housing 21. The transmission element 22 cannot slide out from the two ends of the accommodating housing 21. The transmission element 22 is always kept in the accommodating housing 21. That is, a maximum sliding distance of the two ends of the transmission element 22 in the accommodating housing does not go beyond the two ends of the accommodating housing 21. If either end of the transmission element 22 slides out of the accommodating housing 21, the entire connecting structure will be damaged and cannot be used multiple times.

Further, the inner wall of the accommodating housing 21 is provided with a locking groove.

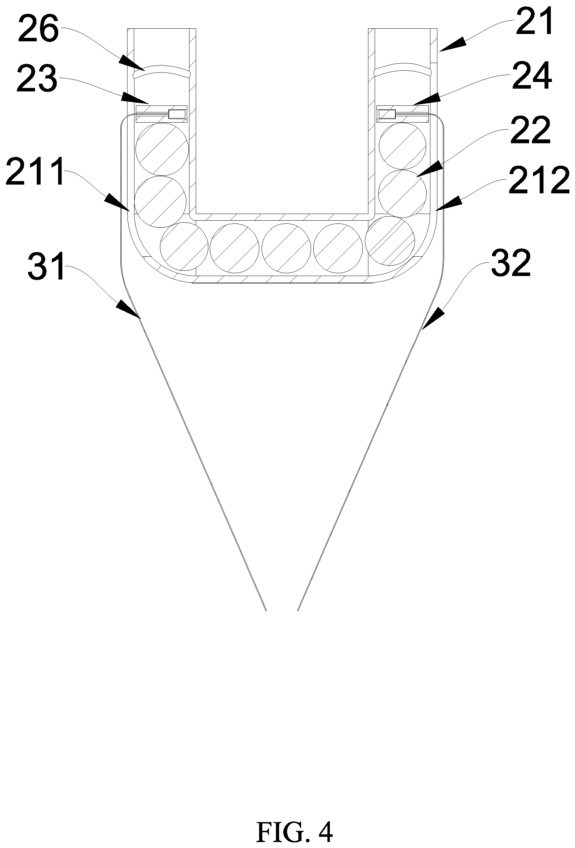

In the above solution, as shown in FIG. 4, the locking groove 26 is provided at a position close to two ends of the inner wall of the accommodating housing 21. The locking groove 26 can be provided at two ends or one end of the accommodating housing 21. Due to the locking groove 26 on the inner wall of the accommodating housing 21, a component connected to the accommodating housing 21 is matched with the locking groove 26 to achieve locking of the component in the accommodating housing 21, avoiding the movement of the component in the accommodating housing beyond a preset position.

Further, limiting portions are respectively provided at the two ends of the accommodating housing 21.

In the above solution, end surfaces or inner walls of the two ends of the accommodating housing 21 are provided with the limiting portions. The limiting portions can be protrusions. A maximum size of the limiting portions is smaller than a maximum size of the transmission element 22, to prevent the transmission element 22 from moving out of the accommodating housing 21 when moving in the accommodating housing 21, thereby limiting the transmission element 22 inside the accommodating housing 21.

In addition, the limiting portions at the two ends of the accommodating housing 21 can also be reduced portions. A cross-sectional size of the reduced portion is smaller than the maximum size of the transmission element 22, in order to limit the transmission element 22 inside the accommodating housing 21.

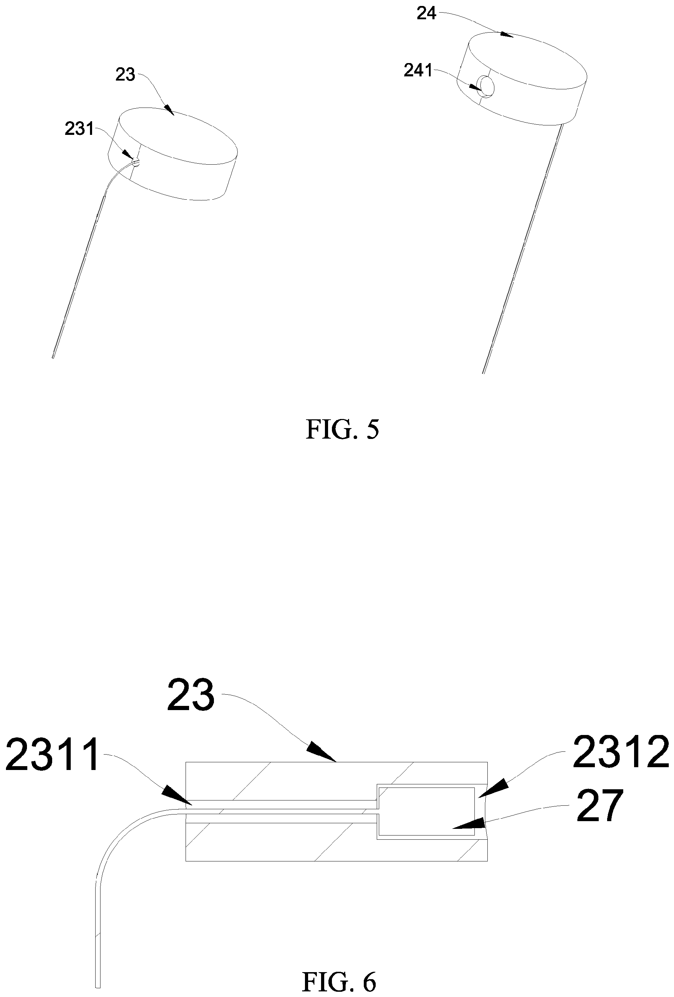

As shown in FIGS. 4 to 7, in the endoscope connecting structure of the present application, the first traction wire mounting element 23 is provided with threading holes 231. The threading holes 231 include first threading hole 2311 and second threading hole 2312. The first threading hole 2311 and the second threading hole 2312 are communicated with each other. A step surface is formed at a junction of the first threading hole 2311 and the second threading hole 2312. First expansion portion 27 is fixedly connected to one end of the first traction wire 31. The first expansion portion 27 is movably provided in the second threading hole 2312. One end of the first expansion portion 27 is abutted against the step surface.

The first traction wire mounting element 23 is cylindrical in shape to facilitate smooth rotation of the first traction wire mounting element 23 in the accommodating housing 21. One end of the first traction wire 31 enters the first traction wire mounting element 23 through the threading hole 231 and does not slide out of the threading hole 231.

It should be noted that in this embodiment, the first traction wire mounting element 23 can also be a quadrangular prism, a triangular prism, or other prims. The shape of the first traction wire mounting element is not limited herein, as long as the first traction wire mounting element 23 is rotatable in the accommodating housing 21.

There can be one, two, or three threading holes 231, which is not limited herein.

In this embodiment, there are two threading holes 231, namely the first threading hole 2311 and the second threading hole 2312. The first threading hole 2211 and the second threading hole 2212 are coaxially arranged and communicated with each other. In this embodiment, the first threading hole 2311 and the second threading hole 2312 are circular. A diameter of the first threading hole 2311 is smaller than a diameter of the second threading hole 2312, thus forming the step surface at the junction of the first threading hole and the second threading hole.

The first threading hole 2311 and the second threading hole 2312 can be in other shapes such as circular, square, triangular, and polygonal, which is not limited herein. The cross-sectional sizes of the first threading hole 2311 and the second threading hole 2312 are different, and the sizes can be areas, radii, widths, or lengths, as long as the step surface can be formed at the junction of the first threading hole and the second threading hole.

The first expansion portion 27 is cylindrical, and includes a solid main body and a hollow tube body. In the present disclosure, the first expansion portion 27 is hollow and cylindrical, and the first expansion portion 27 is cylindrical and topless. One end of the first traction wire 31 enters the first expansion portion 27 through a hole of the first expansion portion 27. An operator can pull out the first traction wire 31 from a topless side of the first expansion portion 27 and tie the end of the first traction wire 31 such that the end of the first traction wire 31 cannot be pulled out of the first expansion portion 27.

The first expansion portion 27 is located in the second threading hole 2312. The end of the first expansion portion 27 connected to the first traction wire 31 is abutted against the step surface, such that the first expansion portion 27 will not enter the first threading hole 2311. Therefore, when the first expansion portion 27 is provided, a maximum width of a cross-section of the first expansion portion 27 is not less than the diameter of the first threading hole 2311.

When the first expansion portion 27 is solid, one end of the first traction wire 31 can be fixedly connected to the first expansion portion 27 through adhesive boding or integrated molding.

It should be noted that the first expansion portion 27 can be in other shapes such as cylindrical, rectangular, cubic, and spherical. The specific shape of the first expansion portion is not limited herein as long as the first expansion portion 27 does not slide out of a hole in the step surface and the first traction wire 31 is always connected to the first traction wire mounting element 23.

As shown in FIGS. 4 to 7, in the endoscope connecting structure of the present application, the second traction wire mounting element 24 is provided with threading holes 241. The threading holes 241 include first threading hole 2411 and second threading hole 2412. The first threading hole 2411 and the second threading hole 2412 are communicated with each other. A step surface is formed at a junction of the first threading hole 2411 and the second threading hole 2412. Second expansion portion 28 is fixedly connected to one end of the second traction wire 32. The second expansion portion 28 is movably provided in the second threading hole 2412. One end of the second expansion portion 28 is abutted against the step surface.

The second traction wire mounting element 24 is cylindrical in shape to facilitate smooth rotation of the second traction wire mounting element 24 in the accommodating housing 21. One end of the second traction wire 32 enters the second traction wire mounting element 24 through the threading hole 241 and does not slide out of the threading hole 241.

It should be noted that in this embodiment, the second traction wire mounting element 24 can also be a quadrangular prism, a triangular prism, or other prims. The shape of the second traction wire mounting element is not limited herein, as long as the second traction wire mounting element 24 is rotatable in the accommodating housing 21.

There can be one, two, or three threading holes 241, which is not limited herein.

In this embodiment, there are two threading holes 241, namely the first threading hole 2411 and the second threading hole 2412. The first threading hole 2411 and the second threading hole 2412 are coaxially arranged and communicated with each other. In this embodiment, the first threading hole 2411 and the second threading hole 2412 are circular. A diameter of the first threading hole 2411 is smaller than a diameter of the second threading hole 2412, thus forming the step surface at the junction of the first threading hole and the second threading hole.

The first threading hole 2411 and the second threading hole 2412 can be in other shapes such as circular, square, triangular, and polygonal, which is not limited herein. The cross-sectional sizes of the first threading hole 2411 and the second threading hole 2412 are different, and the sizes can be areas, radii, widths, or lengths, as long as the step surface can be formed at the junction of the first threading hole and the second threading hole.

The second expansion portion 28 is cylindrical, and includes a solid main body and a hollow tube body. In the present disclosure, the second expansion portion 28 is hollow and cylindrical, and the second expansion portion 28 is cylindrical and topless. One end of the second traction wire 32 enters the second expansion portion 28 through a hole of the second expansion portion 28. An operator can pull out the second traction wire 32 from a topless side of the second expansion portion 28 and tie the end of the second traction wire 32 such that the end of the second traction wire 32 cannot be pulled out of the second expansion portion 28.

The second expansion portion 28 is located in the second threading hole 2412. The end of the second expansion portion 28 connected to the second traction wire 32 is abutted against the step surface, such that the second expansion portion 28 will not enter the first threading hole 2411. Therefore, when the second expansion portion 28 is provided, a maximum width of a cross-section of the second expansion portion 28 is not less than the diameter of the first threading hole 2411.

When the second expansion portion 28 is solid, one end of the second traction wire 32 can be fixedly connected to the second expansion portion 28 through adhesive boding or integrated molding.

It should be noted that the second expansion portion 28 can be in other shapes such as cylindrical, rectangular, cubic, and spherical. The specific shape of the second expansion portion is not limited herein as long as the second expansion portion 28 does not slide out of a hole in the step surface and the second traction wire 32 is always connected to the second traction wire mounting element 24.

### Embodiment 2

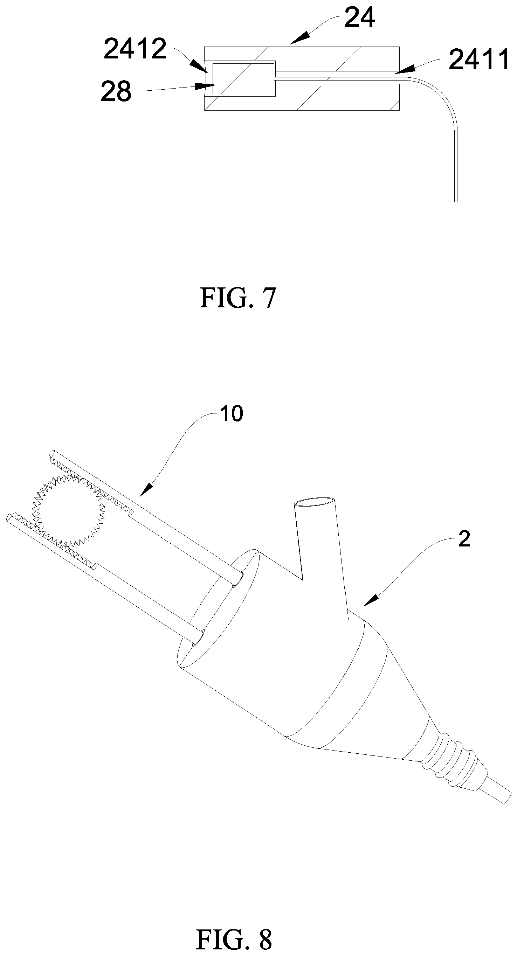

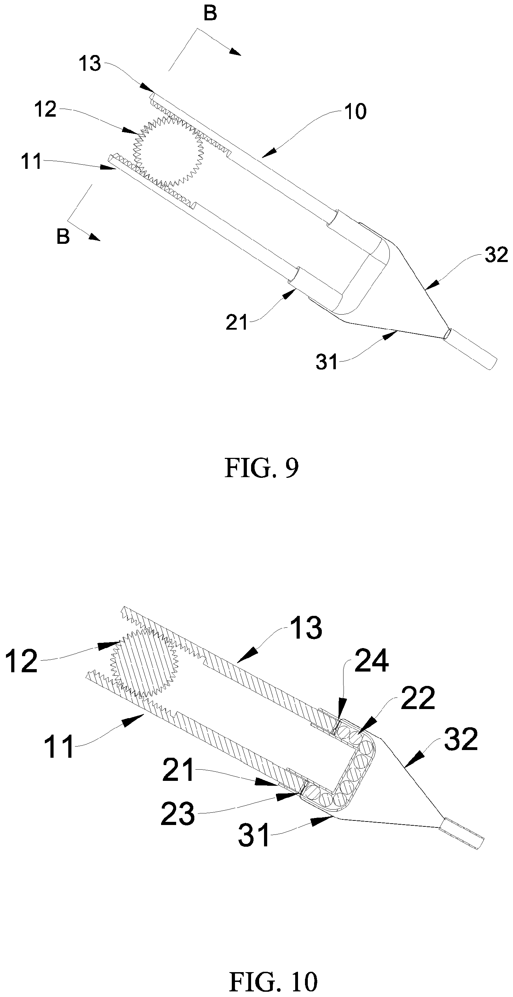

As shown in FIGS. 8 to 10, Embodiment 2 of the present disclosure provides an endoscope handle, including an upper handle and a lower handle.

The upper handle includes pushing assembly 10.

The lower handle includes the above-mentioned endoscope connecting structure.

One end of the pushing assembly 10 is located in the accommodating space and abutted against the traction wire mounting elements 23 and 24.

In the above solution, the endoscope handle includes the upper handle and the lower handle 2. The pushing assembly 10 is at least partially provided in the upper handle. The pushing assembly 10 includes one end located in the upper handle and the other end located in the accommodating space of the accommodating housing 21 of the lower handle and abutted against the traction wire mounting elements 23 and 24 to push the traction wire mounting elements 23 and 24 to slide in the accommodating space.

The pushing assembly 10 is in surface contact, line contact or point contact, etc. with the traction wire mounting elements 23 and 24.

Optionally, the upper handle and the lower handle 2 are detachably or fixedly connected.

Further, the pushing assembly 10 includes at least first pushing element 11, second pushing element 13, and driving element 12.

One end of the first pushing element 11 is abutted against the traction wire mounting elements 23 and 24, and one end of the second pushing element 12 is abutted against the traction wire mounting elements 23 and 24.

The driving element 12 is configured to drive the first pushing element 11 and the second pushing element 13 to move in opposite directions synchronously.

In the above solution, the first pushing element 11 and the second pushing element 13 are racks, and the driving element 12 is provided between the first pushing element and the second pushing element. Optionally, the driving element is a gear. The first pushing element, the second pushing element, and the driving element are engaged. The driving element 12 rotates to drive the first pushing element 11 and the second pushing element 13 to move. In the present application, a movement distance of the transmission element 22 is controllable based on a length of the first pushing element 11 and the second pushing element 13. Alternatively, the movement distance of the rotating element 22 is controllable based on a rotation circumference of the driving element 12.

Optionally, the first pushing element 11 and the second pushing element 13 can also be rods, and the driving element 12 is an electric motor. The present application does not impose any limit on the driving structures of the first pushing element 11, the second pushing element 13, and the driving element 12, as long as they can achieve relative movement.

Specifically, lower ends of the first pushing element 11 and the second pushing element 13 can push the traction wire mounting elements 23 and 24 through point contact, line contact, and surface contact.

In the endoscope handle provided by the present application, the pushing assembly 10 adopts a tooth engagement pushing method to achieve synchronous movement of the first pushing element and the second pushing element. When one pushing element moves down a preset distance, the other pushing element synchronously moves up an equal preset distance. In this way, the operator can control the movement distance of the traction wire by controlling the movement distance of the pushing element, in order to accurately control the traction wire.

Further, a locking element matched with the locking groove is provided on an outer wall of the first pushing element 11 and/or an outer wall of the second pushing element 13.

In the above solution, as shown in FIG. 4, the first pushing element 11 and the second pushing element 13 each are provided with a locking element matched with the locking groove. Alternatively, only one of the first pushing element 11 and the second pushing element 13 is provided with a locking element.

The locking element can be a protrusion provided on the outer wall of the first pushing element 11 and the second pushing element 13, or a combination of a spring and a ball. The present application does not limit the specific structure of the locking element, as long as the locking element is matched with the locking groove to achieve locking of the first pushing element 11 and/or the second pushing element 13 in the accommodating housing. The design avoids the first pushing element 11 and/or the second pushing element 13 from pushing out the transmission element 22 or the traction wire mounting elements 23 and 24 in the accommodating housing, thereby avoiding device damage.

The accommodating housing 21 is fixedly provided in a housing of the lower handle 2.

### Embodiment 3

A third aspect of the present disclosure provides an endoscope, including the above-mentioned endoscope handle.

An inserting unit of the endoscope is connected to the endoscope handle to achieve a function of the endoscope.

## Claims

1. An endoscope connecting structure, comprising:
an accommodating housing (21), provided therein with an accommodating space;
a transmission element (22), slidably provided in the accommodating space;
traction wire mounting elements (23, 24), respectively provided at two ends of the transmission element (22) and located in the accommodating space; and
traction wires (31, 32), wherein one end of each of the traction wires (31, 32) is fixedly connected to a corresponding one of the traction wire mounting elements (23, 24); **characterized in that**
the accommodating housing (21) is U-shaped, V-shaped or W-shaped and provided with a hollow structure; the hollow structure of the accommodating housing (21) forms the accommodating space; the transmission element (22) is slidable and rollable along an inner wall of the accommodating space;
each of the two arms of the accommodating housing (21) is provided at its external side with a wire passage (211, 212); and the traction wires (31, 32) are respectively connected to the traction wire mounting elements (23, 24) through the wire passages (211, 212).

2. The endoscope connecting structure according to claim 1, **characterized in that** the transmission element (22) is either a solid or a viscous liquid; and a viscosity of the viscous liquid is greater than a preset viscosity.

3. The endoscope connecting structure according to claim 1, **characterized in that** the two ends of the transmission element (22) are not higher than two ends of the accommodating housing (21).

4. The endoscope connecting structure according to claim 1, **characterized in that** an inner wall of the accommodating housing (21) is provided with a locking groove.

5. The endoscope connecting structure according to one of claims 1 to 4, **characterized in that** limiting portions are respectively provided at the two ends of the accommodating housing (21).

6. An endoscope handle, **characterized by** comprising:
an upper handle, comprising a pushing assembly (10); and
a lower handle, comprising the endoscope connecting structure according to one of claims 1 to 5, wherein
one end of the pushing assembly (10) is located in the accommodating space and abutted against the traction wire mounting elements (23, 24).

7. The endoscope handle according to claim 6, **characterized in that** the pushing assembly (10) comprises at least a first pushing element (11), a second pushing element (13), and a driving element (12);
one end of the first pushing element (11) is abutted against a first one of the traction wire mounting elements (23, 24), and one end of the second pushing element (13) is abutted against a second one of the traction wire mounting elements (23, 24); and
the driving element (12) is configured to drive the first pushing element (11) and the second pushing element (13) to move in opposite directions synchronously.

8. The endoscope handle according to claim 7, **characterized in that** a locking element matched with the locking groove is provided on an outer wall of the first pushing element (11) and/or an outer wall of the second pushing element (13).

9. An endoscope, **characterized by** comprising the endoscope handle according to one of claims 6 to 8.

## Patentansprüche

1. Endoskop-Verbindungsstruktur, umfassend:
eine Unterbringungsaufnahme (21), die darin mit einem Unterbringungsraum versehen ist;
ein Antriebselement (22), das verschiebbar im Unterbringungsraum bereitgestellt ist;
Zugdraht-Befestigungselemente (23, 24), die jeweils an zwei Enden des Antriebselements (22) bereitgestellt und im Unterbringungsraum befindlich sind, und
Zugdrähte (31, 32), wobei ein Ende eines jeden der Zugdrähte (31, 32) fix mit einem entsprechenden der Zugdraht-Befestigungselemente (23, 24) verbunden ist, **dadurch gekennzeichnet, dass**
die Unterbringungsaufnahme (21) U-förmig, V-förmig oder W-förmig und mit einer hohlen Struktur versehen ist, wobei die hohle Struktur der Unterbringungsaufnahme (21) den Unterbringungsraum bildet, wobei das Antriebselement (22) entlang einer Innenwand des Unterbringungsraums verschiebbar und rollbar ist,
ein jeder der beiden Arme der Unterbringungsaufnahme (21) an seiner externen Seite mit einem Drahtdurchgang (211, 212) versehen ist und die Zugdrähte (31, 32) jeweils mit den Zugdraht-Befestigungselementen (23, 24) durch die Drahtdurchgänge (211, 212) verbunden sind.

2. Endoskop-Verbindungsstruktur nach Anspruch 1, **dadurch gekennzeichnet, dass** das Antriebselement (22) entweder ein Feststoff oder eine visköse Flüssigkeit ist und eine Viskosität der viskösen Flüssigkeit größer als eine voreingestellte Viskosität ist.

3. Endoskop-Verbindungsstruktur nach Anspruch 1, **dadurch gekennzeichnet, dass** die beiden Enden des Antriebselements (22) nicht höher als zwei Enden der Unterbringungsaufnahme (21) sind.

4. Endoskop-Verbindungsstruktur nach Anspruch 1, **dadurch gekennzeichnet, dass** eine Innenwand der Unterbringungsaufnahme (21) mit einer Verriegelungsrille versehen ist.

5. Endoskop-Verbindungsstruktur nach einem der Ansprüche 1 bis 4, **dadurch gekennzeichnet, dass** Begrenzungsabschnitte jeweils an den beiden Enden der Unterbringungsaufnahme (21) bereitgestellt sind.

6. Endoskop-Handgriff, **dadurch gekennzeichnet, dass** er Folgendes umfasst:
einen oberen Handgriff, umfassend eine Drückanordnung (10) und
einen unteren Handgriff, umfassend die Endoskop-Verbindungsstruktur nach einem der Ansprüche 1 bis 5, wobei
ein Ende der Drückanordnung (10) im Unterbringungsraum befindlich ist und an die Zugdraht-Befestigungselemente (23, 24) anschlägt.

7. Endoskop-Handgriff nach Anspruch 6, **dadurch gekennzeichnet, dass** die Drückanordnung (10) mindestens ein erstes Drückelement (11), ein zweites Drückelement (13) und ein Steuerungselement (12) umfasst,
wobei ein Ende des ersten Drückelements (11) an ein erstes Ende der Zugdraht-Befestigungselemente (23, 24) anschlägt und ein Ende des zweiten Drückelements (13) an ein zweites Ende der Zugdraht-Befestigungselemente (23, 24) anschlägt und
das Steuerungselement (12) ausgelegt ist, um das erste Drückelement (11) und das zweite Drückelement (13) so zu steuern, dass diese sich synchron in entgegengesetzte Richtungen bewegen.

8. Endoskop-Handgriff nach Anspruch 7, **dadurch gekennzeichnet, dass** ein passend zur Verriegelungsrille ausgebildetes Verriegelungselement auf einer Außenwand des ersten Drückelements (11) und/oder einer Außenwand des zweiten Drückelements (13) bereitgestellt ist.

9. Endoskop, **dadurch gekennzeichnet, dass** es den Endoskop-Handgriff nach einem der Ansprüche 6 bis 8 umfasst.

## Revendications

1. Structure de raccordement d'endoscope, **caractérisée en ce qu'**elle comprend :
un boîtier de réception (21), pourvu à l'intérieur de celui-ci d'un espace de réception ;
un élément de transmission (22), disposé de manière coulissante dans l'espace de réception ;
des éléments de montage de fils de traction (23, 24), respectivement disposés au niveau de deux extrémités de l'élément de transmission (22) et situés dans l'espace de réception ; et
des fils de traction (31, 32), dans laquelle une extrémité de chacun des fils de traction (31, 32) est reliée de manière fixe à un élément correspondant parmi les éléments de montage de fils de traction (23, 24) ; **caractérisée en ce que**
le boîtier de réception (21) est en forme de U, en forme de V ou en forme de W et pourvu d'une structure creuse ; la structure creuse du boîtier de réception (21) forme l'espace de réception ; l'élément de transmission (22) peut coulisser et rouler le long d'une paroi interne de l'espace de réception ;
chacun des deux bras du boîtier de réception (21) est pourvu au niveau d'un côté extérieur d'un passage de fil (211, 212) ; et les fils de traction (31, 32) sont respectivement reliés aux éléments de montage de fils de traction (23, 24) à travers les passages de fil (211, 212).

2. Structure de raccordement d'endoscope selon la revendication 1, **caractérisée en ce que** l'élément de transmission (22) est soit un solide soit un liquide visqueux ; et une viscosité du liquide visqueux est supérieure à une viscosité prédéfinie.

3. Structure de raccordement d'endoscope selon la revendication 1, **caractérisée en ce que** les deux extrémités de l'élément de transmission (22) ne sont pas supérieures à deux extrémités du boîtier de réception (21).

4. Structure de raccordement d'endoscope selon la revendication 1, **caractérisée en ce qu'**une paroi interne du boîtier de réception (21) est pourvue d'une rainure de verrouillage.

5. Structure de raccordement d'endoscope selon l'une des revendications 1 à 4, **caractérisée en ce que** des parties de limitation sont respectivement disposées aux deux extrémités du boîtier de réception (21).

6. Poignée d'endoscope, **caractérisée en ce qu'**elle comprend :
une poignée supérieure, comprenant un ensemble de poussée (10) ; et
une poignée inférieure, comprenant la structure de raccordement d'endoscope selon l'une des revendications 1 à 5, dans laquelle
une extrémité de l'ensemble de poussée (10) est située dans l'espace de réception et en butée contre les éléments de montage de fils de traction (23, 24).

7. Poignée d'endoscope selon la revendication 6, **caractérisée en ce que** l'ensemble de poussée (10) comprend au moins un premier élément de poussée (11), un deuxième élément de poussée (13) et un élément d'entraînement (12) ;
une extrémité du premier élément de poussée (11) est en butée contre un premier parmi les éléments de montage de fils de traction (23, 24), et une extrémité du deuxième élément de poussée (13) est en butée contre un deuxième parmi les éléments de montage de fils de traction (23, 24) ; et
l'élément d'entraînement (12) est configuré pour entraîner le premier élément de poussée (11) et le deuxième élément de poussée (13) à se déplacer dans des directions opposées de manière synchrone.

8. Poignée d'endoscope selon la revendication 7, **caractérisée en ce qu'**un élément de verrouillage adapté à la rainure de verrouillage est disposé sur une paroi externe du premier élément de poussée (11) et/ou une paroi externe du deuxième élément de poussée (13).

9. Endoscope, **caractérisé en ce qu'**il comprend la poignée d'endoscope selon l'une des revendications 6 à 8.
